Europäisches Patentamt

European Patent Office

Office européen des brevets

(19)

(11) EP 0 886 139 A1

(12) **EUROPEAN PATENT APPLICATION**

(43) Date of publication:
23.12.1998 Bulletin 1998/52

(51) Int. Cl.⁶: **G01N 33/487**, G01N 33/84, C12Q 1/40

(21) Application number: 98110824.4

(22) Date of filing: 12.06.1998

(84) Designated Contracting States:
AT BE CH CY DE DK ES FI FR GB GR IE IT LI LU MC NL PT SE
Designated Extension States:
AL LT LV MK RO SI

(30) Priority: 13.06.1997 JP 171181/97

(71) Applicant:
ORIENTAL YEAST CO., LTD.
Tokyo (JP)

(72) Inventors:
• Hirata, Kimiko
  Takatsuki-shi, Osaka-fu (JP)
• Fujita, Tuyosi
  Ikeda-shi, Osaka-fu (JP)

(74) Representative:
Kern, Ralf M., Dipl.-Ing.
Hansastrasse 16/II.
80686 München (DE)

(54) **A reagent for measurement and a measurement method**

(57) The present invention relates to a reagent for measurement used in a method using absorbance at a wavelength in the range of 400 nm to 450 nm in measuring an enzyme, chlorine ions or calcium ions in serum, characterized in that said reagent comprises hydroxylamine, and by the presence of hydroxylamine, an enzyme such as amylase etc. or various ions in serum can be determined accurately without undergoing any influence of hemoglobin.

FIG. 1

PREVENTION OF THE INFLUENCE IN A SAMPLE CONTAINING HEMOGLOBIN 1000 mg/dL BY HYDROXYLAMINE HYDROCHLORIDE

CONCENTRATION (mM) OF HYDROXYLAMINE IN R1

EP 0 886 139 A1

**Description**

DETAILED DESCRIPTION OF THE INVENTION

Technical Field to Which the Invention Belongs

The present invention relates to a reagent for measurement and a measurement method used in measuring a target substance (also referred to hereinafter as specific substance) in serum, which is medically useful in clinical examination etc.

Prior Art

Conventionally, measurement of enzyme activity and specific ions in serum is used as being responsible for an important role in medical diagnosis. For example, one method of measuring enzyme activity makes use of a substrate having a pigment bound to a certain substance, which comprises dissociating its binding by the action of a target enzyme, measuring the amount of the pigment thus released with time by determining the absorbance, and expressing the enzyme activity in terms of the rate of increase in the absorbance determined per unit time. To determine amylase activity as the target enzyme in serum, for example, a conventional method of measuring amylase in serum uses, as the substrate, a compound having a polysaccharide or amino acid bound to a substance having absorbance at 400 to 450 nm, such as 4-nitrophenol group, 2-chloro-4-nitrophenol group, 4-nitroaniline group, 3-carboxy-4-nitroaniline group, p-nitrophenol group, 6-dichloro-4-nitrophenol group etc.

Known substrates for use in determining amylase activity or specific ions include e.g. 2-chloro-4-nitrophenyl-$\alpha$-D-maltotrioside (hereinafter, abbreviated to G3CNP), 4-nitrophenyl-$\alpha$-D-maltotrioside (hereinafter, abbreviated to G3PNP), 2-chloro-4-nitrophenyl-$\alpha$-D-galactosylmaltoside (hereinafter, abbreviated to GalG2CNP) etc..

In the case where amylase activity or specific ions are determined using G3CNP as the substrate, the activity and concentration thereof are calculated by determining the rate of increase with time of the absorbance, at a wavelength ranging 400 nm to 450 nm, of 2-chloro-4-nitrophenol generated from G3CNP, that is, the rate of increase of the absorbance. This measurement method is called rate assay. Measurement of specific ions is based on the fact that amylase activity increases in proportion to the concentration of specific ions. More specifically, measurement of amylase activity is as follows:

Enzyme activity (IU/L) is determined from the result of measurement of change (increase) with time in absorbance. The enzyme activity of 1 IU/L is defined as the amount of an enzyme catalyzing reaction of 1 $\mu$mol substrate for 1 min., and the enzyme activity (IU/L) from change with time in absorbance (E) is determined by calculating change per min. in absorbance ($\Delta$E/min.) from change with time in absorbance and then introducing this change into the following equation (1):

$$\text{Enzyme activity (IU/L)} = \{[(\Delta E/\text{min.}) \times (RV+SV)]/(\varepsilon \times SV)\} \times 10^{6} \tag{1}$$

RV: Reagent (solution) volume used in measurement.
SV: Sample volume used in measurement.
$\varepsilon$: Molecular extinction coefficient of a substance whose absorbance is measured.

In the method of measuring amylase activity by use of G3CNP as the substrate, it is general that potassium thiocyanate is used in a large amount. The disadvantage of this method is the phenomenon in which a significant negative error is caused in measured amylase activity because of hemolysis action occurring upon preparation of serum (Nippon Rinsho Kensa Jido Kagaku Kaishi, Vol. 9, Complementary Volume, page 141 (1984)). This phenomenon is as a matter of course due to the fact that hemoglobin and its derivatives generated by hemolysis action are gradually decomposed by light or heat in a measurement reagent and further possibly due to the fact that potassium thiocyanate present in a large amount in the reaction solution acts on methohemoglobin to cause a decrease with time in absorbance at a wavelength in the range of 400 nm to 450 nm.

As a result, the rate of increase in absorbance at a wavelength in the range of 400 nm to 450 nm used in measurement of amylase activity will drop to cause negative errors in the measured amylase activity.

Problem to Be Solved by the Invention

In the measurement of absorbance at a wavelength in the range of 400 nm to 450 nm used in the prior art, the object of the present invention is to prevent the phenomenon in which amylase activity is lowered by hemoglobin and its derivatives generated by hemolysis action.

Means to Solve the Problem

The present inventors found that the phenomenon in which enzyme activity is lowered by hemoglobin and its derivatives generated by hemolysis action can be prevented by using a small amount of hydroxylamine in the reagent. That is, the present inventors found that by using hydroxylamine in a test of measuring a specific substance in serum, inert methohemoglobin having no ability to bind to oxygen can be rapidly converted into active hemoglobin having the ability to bind to oxygen so that the phenomenon of lowering enzyme activity by hemoglobin and its derivatives generated by hemolysis action can be prevented even in the presence of potassium thiocyanate, and as a result of their extensive analysis and experiments, the present inventors arrived at the completion of the present invention.

The present invention provides a reagent for measurement used in a measurement method using absorbance at a wavelength in the range of 400 nm to 450 nm in determining a target substance in serum, characterized in that said reagent comprises hydroxylamine.

In the present invention, the specific substance refers to a target substance to be measured, and includes enzymes such as amylase etc. and chlorine ions, calcium ions etc.

The present invention also provides a method of measuring a specific substance in serum by use of the above-described measurement reagent. Specifically, the present invention provides a method of measuring a target substance in serum without undergoing the influence of hemoglobin and its derivatives, characterized in that a reagent for measurement containing an enzyme substrate and potassium thiocyanate is allowed to react with serum in the presence of hydroxylamine and the target substance in said serum is determined on the basis of a free coloring group generated upon decomposition of the amylase substrate. Although a hemolysis sample is used directly as the measurement sample, its accurate and easy measurement is made feasible for the first time without undergoing the influence of hemoglobin and its derivatives brought about by conventionally used potassium thiocyanate.

For the purpose of determining amylase activity even in the presence of potassium thiocyanate, the present invention provides a reagent for measurement of enzyme activity comprising at least hydroxylamine and at least one substrate selected from G3CNP and GalG2CNP in a reagent used for measuring enzyme activity in a method of measuring absorbance in the range of 400 nm to 450 nm, and further provides a measurement method using said reagent for measuring enzyme activity.

Brief Description of the Drawings

Fig. 1 shows the relationship between hydroxylamine hydrochloride concentration (mM) and relative activity of amylase (%).

Fig. 2 is a graph showing the effect of preventing the influence of hemoglobin by hydroxylamine hydrochloride.

Detailed Description of the Invention

The present invention is characterized by preventing the influence of hemoglobin and its derivatives derived from a sample on a reagent for measurement of enzyme activity, and as the preventing agent referred to in the present invention, hydroxylamine can be used.

The preventing agent can be used at a concentration of 0.2 mM to 100 mM in a solution of the reagent for measurement of enzyme activity. This concentration range brings about the effect (referred to hereinafter as the preventing effect) of preventing the negative errors caused by hemolysis in the measurement of enzyme activity. The concentration of the preventing agent does not need to be unnecessarily high, and the preventing agent is used preferably at a concentration of 5 to 20 mM.

In the present invention, the optimum concentration of the preventing agent is not particularly limited and can be suitably determined depending on the conditions of serum as a sample, the type of substrate used, and other various factors under the conditions used.

Mention is made of amylase as the enzyme as the subject of the present reagent for measurement of enzyme activity. Although any known substrate may be used as the substrate of said enzyme, it is preferable to employ at least one member selected from the group consisting of G3CNP, G3PNP, and GalG2CNP.

The preparation of the reagent of the present invention and the method of measuring amylase enzyme activity by use of said reagent follow conventional techniques in the prior art except that hydroxylamine is contained as the preventing agent.

Hereinafter, the present invention is described in more detail with reference to Examples, which however are not intend to limit the scope of the present invention.

For hydroxylamine to be used in the present invention, a commercially available hydroxylamine hydrochloride may be used.

Example 1

Hemoglobin (hereinafter abbreviated to Hb in some cases) in a hemolysis sample was measured for its influence on amylase activity at a main wavelength of 415 nm by using G3CNP as the substrate. Then, the effect of 1000 mg/dL hemoglobin was examined for the degree of its prevention in the presence of hydroxylamine hydrochloride at predetermined concentrations. Further, it was confirmed that the effect of hemoglobin at a varying concentration is prevented in the presence of hydroxylamine hydrochloride. The mixing ratio of reagents, reaction conditions etc. are shown below and in Table 1.

Mixing ratio of reagents: Sample : R1 : R2 = 4 μl : 200 μl : 50 μl
Measurement temperature: 37 °C
Preparation of the hemolysis sample:

(1) Control serum:

1.0 ml pure water
9.0 ml liquid control serum I-Wako (Wako Pure Chemical Industries, Ltd.)

(2) Hemolysis (1000 mg/dL) sample:

1.0 ml hemoglobin solution (10,000 mg/dL)
9.0 ml liquid control serum I-Wako (Wako Pure Chemical Industries, Ltd.)

Table 1

| Hb concentrations | | | | | | |
|---|---|---|---|---|---|---|
| (mg/dL) | 0 | 200 | 400 | 600 | 800 | 1000 |
| (1) | 1.0 ml | 0.8 ml | 0.6 ml | 0.4ml | 0.2 ml | - |
| (2) | - | 0.2 ml | 0.4 ml | 0.6 ml | 0.8 ml | 1.0 ml |

R1 and R2 were prepared according to the Lorenz method.
First reagent (R1):

(1) In the absence of hydroxylamine hydrochloride

62.5 MES
11.25 mM potassium thiocyanate
375 mM sodium chloride
6.25 mM potassium acetate
pH 6.55 (25 °C)

(2) In the presence of hydroxylamine hydrochloride

62.5 MES
11.25 mM potassium thiocyanate
375 mM sodium chloride
6.25 mM potassium acetate
0.2 to 10 mM hydroxylamine hydrochloride
pH 6.55 (25 °C)

A series of varying concentrations of hydroxylamine hydrochloride in R1

Table 2

| Concentrations | | | | | | | |
|---|---|---|---|---|---|---|---|
| (mM) | 0 | 0.2 | 0.5 | 1 | 2 | 5 | 10 |
| (1) | 10.0 ml | 9.8 ml | 9.5 ml | 9.0 ml | 8.0 ml | 5.0 ml | 10.0 ml |
| (2) | - | 0.2 ml | 0.5 ml | 1.0 ml | 2.0 ml | 5.0 ml | - |

Second reagent (R2):

5.0 mM citrate monohydrate
11.25 mM G3CNP solution.

Measurement temp.: 37 °C.
Measurement apparatus: Cobath Faller II (phonetic).
Wavelength: 415 nm
Addition of R2: R2 was added 3 minutes after R1 was mixed with a sample.
Measurement time: 1 min. after addition of R2, absorbance was measured for 1 min.

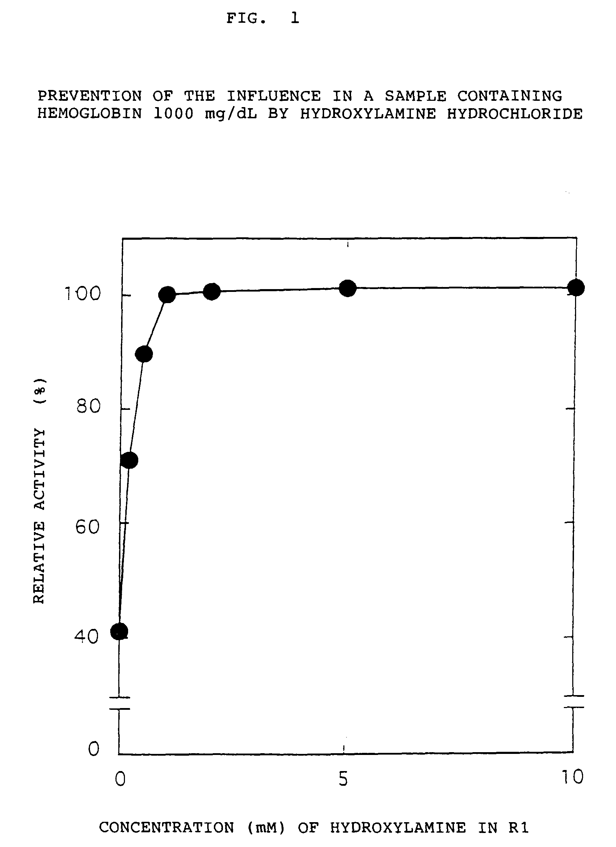

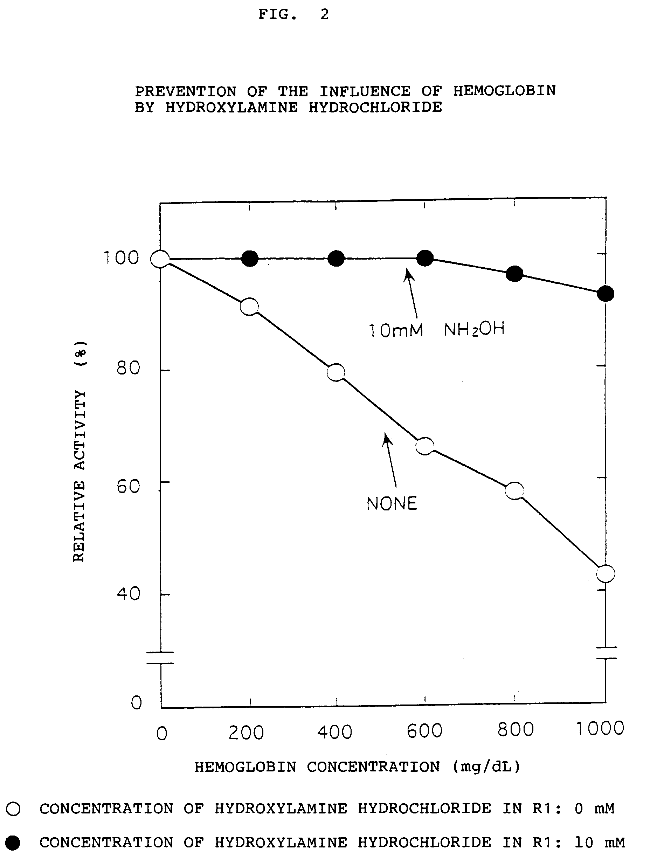

The relationship between hydroxylamine hydrochloride concentration (mM) and relative activity of amylase (%) is shown in Fig. 1.
The effect of hydroxylamine hydrochloride on prevention of the influence of hemoglobin is shown in Fig. 2.

Measurement of amylase activity:

4 μl of the sample was mixed with 200 μl of R1 as said reagent, then the mixture was kept at 37 °C for 3 minutes, and 50 μl of R2 was added to it, and 1 minute after the addition, the absorbance was measured for 1 minute. The increase with time in the absorbance at a main wavelength of 415 nm was measured so that the degree of increase of the absorbance per minute was determined. The determined degree of increase per minute was introduced into the following equation (2) to determine amylase activity.

$$\text{Amylase activity (IU/L)} = [((\text{degree of increase of absorbance per min.}) \times 254)/(14.2 \times 10^3 \times 4)] \times 10^6 \qquad (2)$$

This example showed that hydroxylamine chloride gave effective results.
The results of this test indicated that the influence of hemoglobin by hemolysis on the measurement of amylase activity using C3CNP as the substrate was dissolved by the present invention.

Effect of the Invention

According to the present invention, accurate measurement of an enzyme or various ions is made feasible without undergoing any influence of hemoglobin or its derivatives, so the measurement of such target substances in a hemolysis sample is made feasible for the first time, so the present invention contributes significantly to medicine, particularly clinical examination.

**Claims**

1.  A reagent for measurement used in a method using absorbance at a wavelength in the range of 400 nm to 450 nm for measurement of a target substance in serum, said reagent comprising hydroxylamine.

2.  The reagent for measurement according to claim 1 wherein the target substance is at least one member selected from amylase, chlorine ion and calcium ion.

3.  The reagent for measurement according to claim 1 or 2 wherein amylase activity is measured using at least one substrate selected from 2-chloro-4-nitrophenyl-α-D-maltotrioside, 4-nitrophenyl-α-D-maltotrioside, and 2-chloro-4-nitrophenyl-α-D-galactosylmaltoside

4. A measurement method in which the reagent for measurement as described in claims 1 to 3 containing an enzyme substrate and potassium thiocyanate is allowed to react with serum, and then a target substance in said serum is determined on the basis of a free coloring group generated upon decomposition of the amylase substrate, without undergoing the influence of hemoglobin and its derivatives.

FIG. 1

PREVENTION OF THE INFLUENCE IN A SAMPLE CONTAINING
HEMOGLOBIN 1000 mg/dL BY HYDROXYLAMINE HYDROCHLORIDE

CONCENTRATION (mM) OF HYDROXYLAMINE IN R1

FIG. 2

PREVENTION OF THE INFLUENCE OF HEMOGLOBIN
BY HYDROXYLAMINE HYDROCHLORIDE

10mM NH$_2$OH

NONE

RELATIVE ACTIVITY (%)

HEMOGLOBIN CONCENTRATION (mg/dL)

○ CONCENTRATION OF HYDROXYLAMINE HYDROCHLORIDE IN R1: 0 mM

● CONCENTRATION OF HYDROXYLAMINE HYDROCHLORIDE IN R1: 10 mM

European Patent
Office

**EUROPEAN SEARCH REPORT**

Application Number

EP 98 11 0824

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | DATABASE WPI<br>Section Ch, Week 9408<br>Derwent Publications Ltd., London, GB;<br>Class B04, AN 94-061487<br>XP002900093<br>& JP 06 014799 A (MURAO S)<br>, 25 January 1994<br>* abstract *<br>--- | 1,4 | G01N33/487<br>G01N33/84<br>C12Q1/40 |
| A | US 5 393 660 A (KITAHATA ET AL.)<br>28 February 1995<br>* column 6, line 22 - line 27; claims *<br>--- | 1-3 | |
| A | DATABASE WPI<br>Section Ch, Week 9141<br>Derwent Publications Ltd., London, GB;<br>Class B03, AN 91-300290<br>XP002900094<br>& JP 03 200801 A (KIKKOMAN CORP)<br>, 2 September 1991<br>* abstract *<br>--- | 1-4 | |
| A | DATABASE WPI<br>Section Ch, Week 9018<br>Derwent Publications Ltd., London, GB;<br>Class B04, AN 90-135704<br>XP002900095<br>& JP 02 083391 A (FUJI PHOTO FILM CO LTD)<br>, 23 March 1990<br>* abstract *<br>----- | 1-4 | TECHNICAL FIELDS SEARCHED (Int.Cl.6)<br><br>G01N<br>C12Q |

The present search report has been drawn up for all claims

| Place of search | Date of completion of the search | Examiner |
|---|---|---|
| VIENNA | 11 September 1998 | SCHNASS |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)